# DEMANDE DE BREVET EUROPEEN

(11) **EP 1 138 774 A2**
(43) Date de publication de la demande: **04.10.2001**
(21) Numéro de dépôt: 01115569.4
(22) Date de dépôt: 16.03.1993
(51) Int. Cl.: C12N 15/86, A61K 48/00, C12N 15/19, C12N 15/26, C12N 5/06, A61K 35/12, C07K 14/52, A61P 35/00

(54) **Adénovirus recombinants défectifs exprimant des cytokines pour traitement antitumoral**

(30) Priorité: 16.03.1992 FR 9203120
(62) Demande divisionnaire de: 93920562.1
(71) Demandeur: CENTRE NATIONAL DE LA RECHERCHE SCIENTIFIQUE (CNRS), 75794 Paris Cedex 16 (FR); INSTITUT GUSTAVE ROUSSY, F-94805 Villejuif Cédex (FR)
(72) Inventeur: Haddada, Hédi, 94140 Alfortville (FR); Ragot, Thierry, 92190 Meudon (FR); Perricaudet, Michel, 28150 Ouarville (FR)
(74) Mandataire: Vaillant, Jeanne

(57) **Abrégé**

L'invention concerne un acide nucléique recombinant utilisable pour la production d'un adénovirus défectif contenant un insérat codant pour une cytokine sous le contrôle d'un promoteur au sein de la séquence génomique de l'adénovirus recombinant. Cet adénovirus recombinant est utilisable pour la préparation de médicaments anti-tumoraux sous forme directement injectable dans la tumeur de l'hôte.

## Description

Les cytokines sont des molécules (hormones) produites par des cellules à la suite d'une stimulation antigénique ou d'une activation par d'autres facteurs. La première cytokine qui ait été produite est l'interleukine 1 (IL-1). Elle permet l'activation des cellules T, qui se mettent à leur tour à produire toute une batterie de lymphokines dont certaines sont indispensables pour l'activation du système immunitaire et les défenses contre les infections virales ou parasitaires.

Depuis quelques années, les cytokines sont utilisées en immunothérapie anticancéreuse. Néanmoins leur administration par la voie générale pose un certain nombre de problèmes. L'IL-2 par exemple donne des effet secondaires assez importants; elle est rapidement métabolisée, de sorte que de fortes doses doivent être administrées de façon répétée.

On est donc à la recherche de meilleures voies d'administration qui augmenteraient leur efficacité, tout en diminuant leurs effets indésirables.

L'invention a donc pour objet des adénovirus recombinants défectifs exprimant une ou plusieurs cytokines, ainsi que l'utilisation de ces adénovirus recombinants pour la constitution de compositions pharmaceutiques, notamment antitumorales, plus particulièrement de compositions directement injectables dans des tumeurs solides de l'hôte.

La présente invention a pour objet des adénovirus recombinants défectifs caractérisés en ce qu'ils comportent un génome d'adénovirus non réplicable, défectif dans lequel sont insérées une ou plusieurs séquences d'acide nucléique codant pour une ou plusieurs cytokines, notamment lymphokines, sous le contrôle d'un ou plusieurs promoteurs susceptibles d'être reconnus par les polymérases de cellules humaines, plus particulièrement de cellules tumorales humaines ou de cellules infiltrant es tumeurs.

L'invention concerne plus particulièrement les acides nucléiques recombinants susceptibles d'être mis en oeuvre pour la production de tels adénovirus recombinants défectifs.

Un tel acide nucléique recombinant est caractérisé en ce qu'il comporte, d'une part, une séquence génomique d'un adénovirus défectif en ce qu'elle est dépourvue des séquences nécessaires à sa réplication, mais comportant néanmoins celles des séquences qui dans ce génome sont le support de l'information génétique nécessaire à l'adénovirus correspondant pour pénétrer dans les cellules infectables par celui-ci, ainsi que l'ensemble des séquences essentielles nécessaires à l'encapsidation de cet adénovirus, et, d'autre part, un insérat contenant une séquence nucléique codant pour une cytokine, cet insérat étant sous le contrôle d'un promoteur présent ou préalablement inséré dans la susdite séquence génomique.

Les adénovirus, notamment les adénovirus de type 2 ou 5 susceptibles d'infecter les humains (ou adénovirus humains), ou encore les adénovirus de sérotype 4 et 7, représentent des vecteurs particulièrement préférés dans le cadre de la présente invention, en raison notamment de la grande taille du fragment d'ADN étranger qu'il est possible d'insérer dans le génome de ces virus.

Avantageusement, la ou les séquence(s) d'acide nucléique d'insertion sus-mentionnée(s), codant pour une ou plusieurs cytokines prédéterminées, sont contenues dans un génome défectif d'adénovirus, dépourvu des séquences nucléotidiques essentielles nécessaires à la réplication de ces adénovirus, et plus particulièrement des transactivateurs E1A et E1B et, le cas échéant, de la région E3 de l'adénovirus, ou encore de ses régions E1 et E3.

En d'autres termes l'invention met à profit la capacité de ces adénovirus recombinants défectifs d'autoriser l'expression de la séquence d'insertion qu'ils contiennent dans les cellules qu'ils envahissent, même lorsqu'en raison de leur caractère défectif ils ne s'y multiplient pas. En d'autres termes l'invention a pour objectif de faire secréter les cytokines au sein même des cellules de la tumeur à traiter (cellules tumorales elles-mêmes et cellules, notamment lymphocytes, qui infiltrent ces tumeurs) lorsque celles-ci ont été infectées par ces adénovirus défectifs, en particulier lorsque ceux-ci sont injectés directement dans la tumeur. Les cytokines produites activeront ainsi en priorité, in situ, les cellules cytotoxiques infiltrant la tumeur et celles se trouvant à proximité de la tumeur.

En ce qui concerne la séquence d'insertion dans le génome d'adénovirus recombinant défectif, elle peut être choisie parmi toutes celles qui expriment une cytokine capable d'exercer un effet, soit antitumoral direct, soit activateur de cellules immunocompétentes de l'organisme, soit les deux à la fois.

Parmi ces cytokines on mentionnera à titre d'exemples : IL-1, IL-2, IL-3, IL-4, IL-5, IL-6 interféron α, interféron γ, "tumor necrosis factor" alpha (TNF α) (facteur alpha de nécrose de tumeur).

Les mêmes adénovirus recombinants peuvent aussi être utilisés dans le cas de certaines maladies, où il y a une déficience immunitaire et dans le cas de certaines maladies parasitaires ou virales (en particulierinterféron γ, α et/ou GM-CSF), notamment par administration par la voie générale ou par l'intermédiaire de cellules, de préférence humaines prises dans un état autorisant leur injection chez l'homme, ces cellules ayant au préalable été infectées par un adénovirus défectif recombinant conforme à l'invention.

On rappelle ci-après les propriétés de certaines de ces cytokines.

### Interleukine 1 (IL-1) :

Elle est produite essentiellement par les monocytes et les macrophages activés. Son poids moléculaire est d'environ 17 Kilodaltons. Elle présente plusieurs activités, parmi lesquelles :
**a)** une action chemoattractive sur les cellules polymorphonuclées et les macrophages (1,2),
**b)** une augmentation de l'activité cytotoxique des cellules cytotoxiques spontanées ("Natural Killer", ou Nk),
**c)** une induction de fièvre à la suite d'une infection,
**d)** surtout l'activation des cellules T pour la production d'autres facteurs.

### Interleukine 2 (IL-2), Interleukine 4 (IL-4) et Interleukine 5 (IL-5) :

Elles sont produites par des lymphocytes T activés. L'action de ces cytokines est restreinte aux cellules du sytème immunitaire, elles provoquent leur multiplication et leur activation : IL-2 et IL-4 ont été essayés en immunothérapie antitumorale chez la souris et chez l'homme. Chez la souris, elles agissent de façon synergique et provoquent la régression tumorale.

### Interleukine 6 (IL-6)

Elle est produite par des nombreux types cellulaires dont les lymphocytes T, les macrophages, les fibroblastes... Elle induit la différenciation finale des lymphocytes B qui deviennent producteurs d'anticorps.

### "Tumor Necrosis Factor-α" (TNF-α) (facteur-α de nécrose des tumeurs) :

C'est un facteur produit par les macrophages. Il a une double action : une action directe sur les cellules tumorales en provoquant leur lyse et une activation du sytème immunitaire.

L'utilisation du TNF-α chez l'homme doit se faire avec précaution, étant donné que de nombreuses cellules en possèdent le récepteur : d'où l'intérêt de n'induire sa sécrétion que localement, au sein même de la tumeur, pour limiter ses effets non désirables sur les autres cellules de l'hôte.

### Interleukine 3 (IL-3), Interleukine 7 (IL-7), et "Colony stimulating Factor" (CSF).

Ce sont des facteurs de croissance hématopoiétique. Ils sont produits essentiellement par les lymphocytes, les monocytes et les macrophages. Ils agissent à différents niveaux de l'hématopoïese, c'est-à-dire des différentes étapes de différenciation de cellules de moëlle en cellules sanguines. En outre le CSF exerce des effets très importants au niveau des défenses primaires de l'organisme vis-à-vis des défenses bactériennes en attirant les macrophages vers les sites d'infection et en augmentant leur capacité de phagocytose.

En combinaison avec l'IL-2 et l'IL-4, le GM-CSF, s'avère être un important facteur antitumoral.

### L'interféron γ (IFN γ)

C'est un facteur produit par les cellules T activées; il est doué de propriétés antivirales; il inhibe la multiplication des virus et des parasites et provoque la lyse des cellules infectées et de certaines cellules tumorales.

### L'interféron α(IFNα)

Produit par des cellules T et des monocytes, il présente un effet antiviral et lytique sur des cellules infectées. L'IFN α a été utilisé en immunothérapie contre certains types de cancer dont le mesothélium.

Bien entendu l'invention n'est pas limitée, quant au choix des séquences d'insertion utilisables dans des adénovirus conformes à l'invention, à celles qui ont été identifiées ci-dessus. Néanmoins celles-ci sont illustratives de la palette des possibilités qui s'offrent au thérapeute, à qui appartient le choix de l'adénovirus recombinant défectif le plus approprié à mettre en oeuvre, compte tenu de la nature des tumeurs à combattre.

L'invention concerne également des compositions pharmaceutiques comprenant un ou plusieurs vecteurs recombinants tels que décrits ci-dessus, en association avec un véhicule pharmaceutiquement acceptable, en particulier des compositions directement injectables dans les tumeurs à traiter, stériles, isotoniques, ou des compositions sèches, notamment lyophilisées, qui, par addition selon le cas d'eau stérilisée ou de sérum physiologique, permettent la constitution ou reconstitution de solutés directement injectables dans les tumeurs.

L'injection directe dans la tumeur d'adénovirus modifiés, non réplicables, offre l'avantage d'une part d'éviter la diffusion des adénovirus recombinants dans la circulation générale avec, pour conséquence, les effets secondaires susceptibles d'être exercés par les cytokines exprimées ailleurs que sur les sites où la manifestation de leur action est recherchée, en l'occurence les cellules tumorales, elles-mêmes ou les cellules, notamment lymphocytes, qui les infiltrent ou qui se trouvent à leur proximité immédiate. De préférence, l'injection est réalisée à tout le moins dans au moins un site de la tumeur primaire.

L'invention n'est pas davantage limitée à l'administration des adénovirus recombinants contenant les séquences codant pour les cytokines du genre en question directement dans les tumeurs. Toute autre voie d'administration permettant un accès de ces adénovirus recombinants à la tumeur à traiter peut être envisagée. En particulier, on peut avoir recours à des cellules compatibles avec l'hôte, par exemple des fibroblastes humains, de préférence préalablement prélevés chez l'hôte lui-même.

L'invention concerne également des cultures cellulaires, par exemple des fibroblastes en culture préalablement infectés par des acides nucléiques recombinants, plus particulièrement les adénovirus défectifs sus-définis. Ces cellules infectées, le cas échéant atténuées ou rendues immunologiquement inertes, par exemple par irradiation, contribuent à l'éradication de tumeurs installées, lors de leur injection par la voie générale. Cette injection peut être envisagée soit seule, soit en sus de l'injection directement dans la tumeur.

L'invention a également pour objet un procédé d'obtention des adénovirus recombinants décrits ci-dessus qui comprend, après l'étape de construction proprement dite d'un vecteur par introduction d'un ou plusieurs acide(s) nucléique(s) d'insertion dans le génome de l'adénovirus défectif initial, une étape de transformation de lignées cellulaires transformables d'eucaryotes supérieurs (notamment d'origine humaine ou animale) comportant elles-mêmes une séquence distincte de nucléotides apte à complémenter la ou les parties qui font défaut au génome de l'adénovirus défectif et sans lesquelles la réplication de ce dernier est interdite, ladite séquence distincte étant de préférence incorporée au génome des cellules de ladite lignée cellulaire.

A titre d'exemple préféré de telles lignées cellulaires, on mentionnera la lignée 293, lignée de rein embryonnaire humain qui contient, intégrés dans son génome, les onze premiers pourcents de l'extrémité gauche du génome d'un adénovirus de type 5 (Ad5). Ceux-ci peuvent alors complémenter des virus recombinants défectifs qui portent des délétions de cette région. Un tel procédé d'obtention est plus particulièrement décrit dans la demande de brevet européen n° 0 185 573 du 20/11/85.

Après transformation de ces lignées cellulaires, les adénovirus défectifs ainsi multipliés et produits sont récupérés à partir du milieu de culture des cellules de ces lignées et purifiés.

La présente invention sera d'avantage détaillée dans la description qui suit des possibilités de construction d'un adénovirus vecteur recombinant contenant au moins une séquence codant pour une cytokine, en particulier une lymphokine.

### I. METHODES

### a) Cellules et virus

La lignée cellulaire 293 rénale embryonnaire humaine transformée par Ad-5 (Graham et al., 1977), a été utilisée pour la transfection d'ADN ainsi que pour la multiplication et la titration d'Adénovirus (Ad). En effet, la lignée cellulaire 293 complémente les fonctions des gènes de fonctions E1A et E1B et permet la réplication des Ad-recombinants défectifs. Pour la construction de l'Ad recombinant, l'Ad5-dl324 humain, portant des délétions dans les régions E1 (3.9-10.5 m.u.) et E3 (78.5-84.3 m.u.), a été utilisé (Shenk et Williams, 1984). Les lignées cellulaires 293, Hela et Vero ont été maintenues dans un milieu de culture minimum essentiel Eagle avec 10 % de sérum de veau fétal.

### b) Construction des plasmides permettant l'expression de différentes cytokines

Le vecteur d'expression eucaryote pMLP10 a été décrit (Ballay et al., 1985). Un dérivé de ce vecteur (pMLP18) a été construit par insertion d'une séquence contenant différents sites uniques de restriction en aval du promoteur majeur tardif d'adénovirus. Ces sites permettant ainsi le clonage des différents gènes codant pour les cytokines choisies sous le contrôle du promoteur viral. En aval de cette séquence contenant ces sites uniques de restriction a été placée la séquence contenant le signal de polyadénylation du gène codant pour les antigènes précoces du virus SV40. Le fragment BgIII - HindIII d'Ad5 est cloné en aval. Cette séquence de 3 Kpb contient le gène codant pour la protéine IX qui est nécessaire pour l'encapsidation du génome viral au-delà de 97 % de sa taille normale et permet la recombinaison in vivo ultérieure. Les séquences codant pour les gènes des différentes cytokines sont isolées à partir de plasmides obtenus auprès de différentes équipes. Ces séquences, obtenues après clivage au moyen de différentes enzymes de restriction sont introduites dans le site de clonage multiple du vecteur d'expression décrit ci-dessus (pMLP-18). On obtient ainsi les différents plasmides dénommés pMLP-cytokine (IL-2, IL-4 etc...) qui sont utilisés pour l'obtention des virus recombinants comme décrit dans le paragraphe suivant.

### c) Transfection d'ADN et isolement de virus recombinants

Les adénovirus recombinants défectifs Adcytokines ont été obtenus par recombinaison in vivo entre le fragment droit du génome viral clivé au préalable par l'enzyme de restriction Cla I et la séquence homologue existant sur les plasmides pMLP-cytokine décrits ci-dessus. Le mélange du fragment du génome viral (2,6 m.u. - 100 m.u.) purifié après clivage et du plasmide linéarisé par l'enzyme de restriction Cla I ou Pvu I est transfecté dans les cellules 293 en utilisant la méthode de précipitation au phosphate de calcium (Graham et Van Der Eb, 1973). Des plages de cellules montrant un effet cytopathique ont été isolées 10 jours après et le virus a été amplifié en culture. L'ADN viral a été extrait par la procédure Hirt (Graham et al., 1977) et les virus recombinants ont été identifiés par cartographie avec des enzymes de restriction.

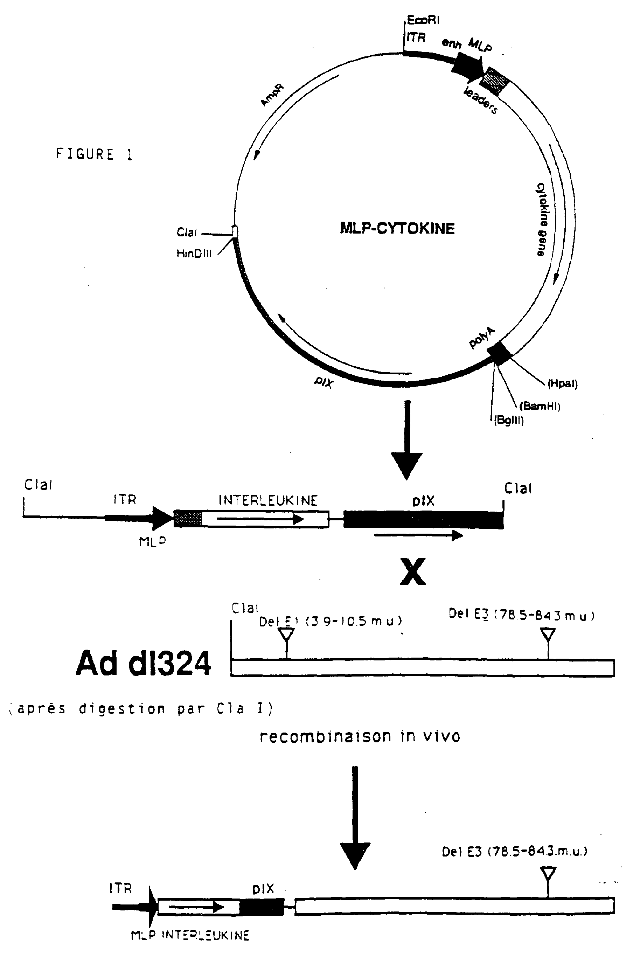

La **figure 1** représente schématiquement une construction de ce type mettant en oeuvre une séquence d'insertion codant pour une interleukine (IL-2, IL-4, etc..).

Dans cette figure :
- "leaders" correspond à un leader tripartite
- "Del" correspond à une "délétion"
- Ad dl 324 correspond à un adénovirus pourvu des "délétions" sus-indiquées.

### d) Expression des séquences codant pour une cytokine exprimée

Des lignées cellulaires Hela ou Vero sont infectées avec les virus recombinants défectifs obtenus. Les cellules effectivement transfectées peuvent être caractérisées essentiellement grâce à la détection de l'activité de la cytokine libérée dans leur milieu de culture. Dans le cas de IL-II des rendements pouvant atteindre de 1 à 2 *µ*g d'interleukine pour 10⁶ cellules ont été observés.

Les cellules infectées par un recombinant Adcytokine sécrètent dans le milieu de culture des quantités variables de la cytokine. Il existe différentes méthodes pour la détection et la quantification des cytokines produites.

### 1) Méthodes quantitatives :

- ELISA, en utilisant des anticorps spécifiques
- RIA (radioimmunoassay)
- Western blot

### 2) Méthodes qualitatives (ou biotests) : basées sur les propriétés biologiques des cytokines

Par exemple :
**IL-2** : Test de prolifération des cellules CTL-L2 (les cellules CTL-L2 ne se multiplient et ne se maintiennent en culture qu'en présence d'IL-2 dans le milieu de culture)
**IL-3 et GM-CSF** : Test de prolifération des cellules TF-1
**IL-4 :** Test de prolifération des cellules CTL-L2 et induction de CD23 solubles par certaines cellules dont les lymphocytes.
**IMF-α** : Test de cytotoxicité sur les cellules L92-9.

**Test de neutralisation** : L'effet des cytokines peut être bloqué en incubant les cellules cibles en présence de cellules d'anticorps spécifiques.

Quelques résultats obtenus avec le vecteur adénovirus portant le gène de l'IL-2(Ad-IL-2) sont exposés ci-après.
**1)** Les cellules infectées in vitro avec l'Ad-IL-2 sécrétant des quantités significatives d'IL-2 fonctionnelle.
**2)** L'injection directe du vecteur portant le gène de l'IL-2 dans des tumeurs déjà établies chez l'animal (le diamètre tumoral au moment de l'injection est entre 4 et 7 mm) induit la stimulation des systèmes immunitaires qui se traduit par une stabilisation de la taille de la tumeur, puis sa régression jusqu'à disparition complète dans 40 % à 50 % des cas.
   Ce résultat peut être amélioré en traitant les tumeurs dans une phase plus précoce de son développement ou en utilisant différents vecteurs à la fois par exemple association de Ad-IL-2 avec Ad-INF et/ou Ad-IL-4, Ad-GM-CSF, Ad-IL-3. Cette combinaison est à préciser selon le type de tumeur.
**3)** Les cellules tumorales infectées in vitro puis injectées à des animaux syngénéiques ou même à des animaux immunodéficients (souris Nu/Nu) perdent leur pouvoir tumorigène (au moins jusqu'à 80 % des animaux rejettent les cellules tumorales ; en d'autres termes, les cellules tumorales ne prolifèrent plus chez 80 % des animaux immunodéficients injectés avec ces cellules.
**4)** Les animaux ayant rejeté une première injection de cellules tumorales infectées sont hautement immunisés et sont protégés contre des cellules tumorales parentales (non infectées) et injectées à différents temps et à différents endroits.

Co-injectées avec des cellules tumorales infectées in vitro, les cellules spléniques de ces animaux immunisés sont en plus capables de transférer l'immunité anti-tumorale à des animaux récipients.

Il va de soi que les descriptions de constructions d'adénovirus défectifs recombinants ci-dessus envisagées n'ont aucun caractère restrictif. D'autres constructions peuvent être réalisées, notamment selon les variantes encore indiquées ci-après à titre d'exemples.

### 1) Echange des promoteurs

Le promoteur majeur tardif d'Adénovirus peut être substitué par d'autres promoteurs ubiquitaires mais d'origine exogène tels que :
- promoteur contenu dans le LTR (Long Terminal Repeat) de RSV (Rous Sarcome Virus)
- le promoteur du gène IE de CMV (cytomégalovirus)
- les promoteurs inductibles MMTV (Mouse Mammary Tumor Virus) ou métallothionine.

De même des promoteurs permettant une expression plus spécifique, restreinte aux cellules tumorales peuvent être utilisés comme par exemple :
- le promoteur du gène rep du parvovirus HI.

L'invention concerne encore un acide nucléique recombinant du type sus-indiqué, caractérisé en ce que la séquence génomique de l'adénovirus est dépourvue de sa région d'extrémité 5', en aval du promoteur précoce de la région E1A de l'adénovirus, et en ce que la séquence codant pour la cytokine est placée sous le contrôle de ce promoteur précoce. Cet acide nucléique recombinant peut également être mis en oeuvre dans les applications plus particulièrement mentionnées en rapport avec les ADNs recombinants dans lesquels la séquence codant pour la cytokine est placée sous le contrôle du promoteur majeur tardif de l'adénovirus.

### 2) Expression simultanée de plusieurs gènes de cytokines

3 types de constructions sont décrites :
- les gènes de cytokines sont sous le contrôle de deux promoteurs soit identiques, soit différents (MLP et RSV par exemple) et situés à la suite l'un de l'autre.
- les gènes des cytokines sont sous le contrôle de promoteurs distincts et clonées dans des régions distinctes du virus.

## Revendications

1. Acide nucléique recombinant comportant, d'une part, une séquence génomique d'un adénovirus défectif en ce qu'elle est dépourvue des séquences nécessaires à sa réplication, mais comportant néanmoins celles des séquences qui dans ce génome sont le support de l'information génétique nécessaire à l'adénovirus correspondant pour pénétrer dans les cellules infectables par celui-ci, ainsi que l'ensemble des séquences essentielles nécessaires à l'encapsidation de cet adénovirus, et, d'autre part, un insérat contenant une séquence nucléique codant pour une cytokine, cet insérat étant sous le contrôle d'un promoteur présent ou préalablement inséré dans la susdite séquence génomique.

2. Acide nucléique recombinant selon la revendication 1 **caractérisé en ce qu'**il est dépourvu des transactivateurs E1A et E1B et, le cas échéant, de la région E3 de l'adénovirus.

3. Acide nucléique recombinant selon la revendication 1 **caractérisé en ce que** la séquence génomique de l'adénovirus est dépourvue de sa région d'extrémité 5', en aval du promoteur précoce de la région E1A de l'adénovirus, et **en ce que** la séquence codant pour la cytokine est placée sous le contrôle de ce promoteur précoce.

4. Acide nucléique recombinant selon la revendication 1 ou la revendication 2 **caractérisé en ce que** la séquence codant pour la cytokine est placée sous le contrôle d'un promoteur tardif de l'adénovirus.

5. Acide nucléique recombinant selon la revendication 1, **caractérisé en ce que** la séquence génomique de l'adénovirus est pourvue d'un promoteur étranger au génome de l'adénovirus, et **en ce que** la séquence codant pour la cytokine est placée sous le contrôle de ce promoteur étranger.

6. Acide nucléique recombinant caractérisé, soit en ce que l'insérat contient des séquences codant pour plusieurs cytokines, soit en ce qu'il contient des insérats distincts respectivement placés sous le contrôle de promoteurs distincts, également distincts.

7. Adénovirus défectif **caractérisé en ce qu'**il contient l'acide nucléique recombinant selon l'une quelconque des revendications 1 à 6.

8. Culture de cellules, notamment d'origine humaine, **caractérisée en ce qu'**elles sont infectées par l'adénovirus selon la revendication 7.

9. Composition pharmaceutique contenant l'adénovirus recombinant selon la revendication 7 en association avec un véhicule pharmaceutiquement acceptable, notamment.

10. Utilisation de l'adénovirus recombinant selon la revendication 7 pour la préparation de médicaments antitumoraux, de préférence sous forme directement injectable dans une tumeur de l'hôte.

11. Composition pharmaceutique contenant des cellules selon la revendication 8, de préférence humaines, dans un état autorisant leur injection à l'homme.

12. Procédé de production d'adénovirus défectifs recombinants selon la revendication 7 **caractérisé par** la transformation de lignées cellulaires transformables d'eucaryotes supérieurs (notamment d'origine humaine ou animale) comportant elles-mêmes une séquence distincte de nucléotides apte à complémenter la partie du génome de l'adénovirus dont celui-ci est dépourvu et qui serait essentielle à sa réplication, ladite séquence distincte étant de préférence incorporée au génome des cellules de ladite lignée cellulaire, et en ce que l'on récupère les adénovirus recombinants défectifs produits à partir du milieu de culture des cellules desdites lignées cellulaires.

13. Procédé selon la revendication 12 **caractérisé en ce que** le génome d'adénovirus défectifs est dépourvu de sa région d'extrémité 5' et que la lignée cellulaire est une lignée de rein embryonnaire humain telle que la lignée 293, qui contient, intégré dans son génome, une région d'extrémité 5' du génome d'un adénovirus de type 5 (Ad5) et ayant une taille correspondante à approximativement 11 % de celle du génome entier de cet adénovirus.
